Europäisches Patentamt

European Patent Office

Office européen des brevets

(19)

(11) Publication number: **0 183 272 B1**

(12)

# EUROPEAN PATENT SPECIFICATION

(45) Date of publication of patent specification: **02.09.92**

(21) Application number: **85115146.4**

(22) Date of filing: **29.11.85**

(51) Int. Cl.⁵: **C12N  15/71**, C12N 15/19, C12N 1/21, //(C12N1/21, C12R1:19)

(54) **Vector capable of efficient gene expression and utilization of such vector.**

(30) Priority: **30.11.84 JP 253423/84**

(43) Date of publication of application:
**04.06.86 Bulletin  86/23**

(45) Publication of the grant of the patent:
**02.09.92 Bulletin  92/36**

(84) Designated Contracting States:
**AT BE CH DE FR GB IT LI LU NL SE**

(56) References cited:
**EP-A- 0 073 657       EP-A- 0 094 797
EP-A- 0 111 814       EP-A- 0 129 268
EP-A- 0 136 490       EP-A- 0 173 280
EP-A- 0 182 636**

**GENE, vol. 28, no. 1, April 1984, pages 55-64,
Elsevier Science Publishers, Brussels, BE;
G. SIMONS et al.: "High-level expression of
human interferon gamma in Escherichia coli
under control of the PL promoter of bac-
teriophage lambda"**

(73) Proprietor: **SUNTORY LIMITED
1-40, Dojimahama 2-chome
Kita-ku, Osaka-shi, Osaka-fu 530(JP)**

(72) Inventor: **Oshima, Takehiro
17-10-383, Besshohonmachi
Takatsuki-shi Osaka(JP)**
Inventor: **Tanaka, Shoji
1-8-46, Minamikaneden-cho
Suita-shi Osaka(JP)**

(74) Representative: **Lehn, Werner, Dipl.-Ing. et al
Hoffmann, Eitle & Partner Patentanwälte Ar-
abellastrasse 4
W-8000 München 81(DE)**

THE EMBO JOURNAL, vol. 1, no. 11, 1982, pages 1399-1404, IRL Press Ltd, Oxford, GB; D. STUEBER et al.: "Transcription from efficient promoters can interfere with plasmid replication and diminish expression of plasmid specified genes"

NUCLEIC ACIDS RESEARCH, vol. 9, no. 8, 1981, pages 1973-1989, IRL Press Ltd, London, GB; B.E. ENGER-VALK et al.: "The construction of new vehicles for the cloning of transcription termination signals"

## Description

Background of the Invention:

The present invention relates to a vector capable of efficient gene expression that is useful in protein or polypeptide production using recombinant DNA technology, and utilization of such vector. More particularly, the present invention relates to a vector capable of efficient gene expression wherein a base sequence including a region having the transcription terminating function is linked immediately downstream from a base sequence encoding the translation termination of a structural gene coding for the protein or polypeptide to be produced. The invention also relates to a process for producing a protein or polypeptide using such a vector.

Many attempts have been made to use the recombinant DNA technology in the production of a useful substance, particularly a physiologically active protein or polypeptide, in microorganisms, animal or plant cells, and th basic techniques for attaining this objective have been substantially established. At the same time, attempts have been made by many researchers toward efficient production of the desired substances. As for expression vectors, improvements and modifications have been made on the promoter region for accelerated transcription, the ribosome-binding site (Shine-Dalgarno sequence) for ensuring efficient translation of mRNA, as well as the base sequence of said ribosome-binding site and the distance between said ribosome-binding site and the translation initiation codon (ATG). All of these efforts are directed to the 5' end non-translational region present upstream from the structural gene to be expressed, and few studies have been made on the 3' end non-translational region with a view to achieving efficient expression of an exogenous gene. It is already known that in eucaryotic cells, the non-translational region downstream from (at the 3' end of) the codon for terminating the translation of a structural gene will improve the efficiency of the production of an exogenous protein or polypeptide (Unexamined Published Japanese Patent Application Nos. 146281/1983 and 74986/1984). However, no case exists where a specific study has been made of the 3' end non-translational region which could affect the efficiency of the production of an exogenous protein or polypeptide using the recombinant DNA technology with procaryotic cells such as E. coli.

The present inventors postulated that not only in eucaryotic host cells but also in procaryotic cells such as E. coli, the 3' end non-translational region would cause some effects on the expression of an exogenous gene incorporated in an expression vector or on the production of an exogenous protein or polypeptide. As a result of various studies made to confirm this assumption, the present inventors found that if a base sequence containing a region having the E. coli trpA terminator is linked immediately downstream from a base sequence encoding the termination of the translation of the structural gene to be expressed, the efficiency of the expression of the desired structural gene, and, hence, the production of the desired protein or polypeptide in E. coli can be significantly improved. The present invention has been accomplished as a consequence of this finding.

The present invention is hereunder described with reference to the case of producing an exogenous polypeptide in E. coli using a chemically synthesized base sequence coding for the polypeptide, and having a human gamma interferon (hereinafter referred to as hIFN-γ) activity as a structural gene, and a base sequence having the trpA transcription terminating function in E. coli

Brief Description of the Drawings:

Fig. 1 is a diagram showing the route by which a chemically synthesized TrpA terminator is introduced into a plasmid vector pIN5GIF54 to construct plasmid vector pIN5A2 in accordance with the present invention;

Fig. 2 is a diagram showing the route for constructing plasmid vector pIN5T8 where Tc$^r$ is substituted for Ap$^r$ as the drug-resistance marker in the plasmid vector pIN5A2 constructed in Fig. 1, as well as the route for constructing plasmid vector pIN5T4 that is equivalent to pIN5T8 except that it is free from the TrpA terminator region;

Fig. 3 is a photomicrograph showing Coomassie blue stained protein bands from SDS-PAGE, including the band for GIF146 produced by cultures of E. coli W3110/pIn5T8 and W3110/pIn5T4 that have been transformed by the plasmid vectors pIN5T8 and pIN5T4, respectively, constructed by the routes shown in Fig. 2;

Fig. 4 is a diagram showing the route for constructing plasmid vector pIN5T5 where that region of Ap$^r$ gene present on pBR322 which is downstream from the PstI cleavage site is linked immediately downstream from the codon for the termination of translation of GIF146 structural gene on pIN5GIF54, and the route for constructing plasmid vector pIN5T13 where the same region of Ap$^r$ is linked downstream from the TrpA terminator of pIN5A2;

Fig. 5 is a diagram showing the route for constructing plasmid vector pIN5T15 where the TrpA terminator is inserted far downstream from the translation terminating codon of the GIF146 structural gene;

Fig. 6-a is a photomicrograph showing the SDS-PAGE patterns for the products obtained by culturing strains of E. coli W3110 transformed with plasmid vectors, pIN5T5, pIN5T13 and pIN5T15, and Fig. 6-b shows traces obtained by gel-scanning the SDS-PAGE protein bands;

Fig. 7 is a diagram showing the route for constructing plasmid vector pIN5T9 where that region of Ap$^r$ gene present on pBR322 which is downstream of the BglI cleavage site is linked immediately downstream from the codon for the termination of translation of GIF146 structural gene on pIN5GIF54, and the route for constructing plasmid vector pIN5T11 where the same region of Ap$^r$ is linked downstream from the TrpA terminator on pIN5A2;

Fig. 8 is a diagram showing the routes for constructing plasmid vectors pIN5T5-131 and pIN5T13-131 where the GIF146 structural gene portions on pIN5T5 and pIN5T13, respectively, are replaced by GIF131 structural gene portions;

Fig. 9 is a diagram showing the routes for constructing plasmid vectors pIN5T11-131 and pIN5T8-131 where the GIF146 structural gene portions on pIN5T11 and pIN5T8, respectively, are replaced by GIF131 structural gene portions; and

Fig. 10-a is a photomicrograph showing the SDS-PAGE patterns for the products obtained by culturing strains of E. coli W3110 transformed with plasmid vectors, pIN5T5-131 and pIN5T13-131, and Fig. 10-b shows traces obtained by gel-scanning the SDS-PAGE protein bands, with untransformed E. coli W3110 being shown as the control in each Figure.

Detailed Description of the Invention:

The present inventors previously constructed an expression vector pIN5GIF54 for the chemically synthesized gene of hIFN-$\gamma$ composed of 146 amino acids (said gene is hereunder abbreviated as GIF146) using an improved promoter of the gene coding for an E. coli coat protein (lipoprotein) (see Japanese Patent Application No. 132524/1983). The present inventors also constructed an expression vector pIN5GIF54-131 for the chemically synthesized gene for hIFN-$\gamma$ analog composed of 131 amino acids (said gene is hereunder abbreviated as GIF131) using the same improved promotor. Using these two vectors, the inventors succeeded in efficient production of GIF146 and GIF131, respectively, in E coli (see Japanese Patent Application Nos. 132524/1983 and 241457/1983). On the basis of this success, the inventors tried to improve the expression vectors by modifying the non-translational region at the 3' end of each synthetic gene.

In the pages that follow are described the construction of the vector of the present invention capable of efficient expression of the gene for human IFN-$\gamma$ as a useful substance, and utilization of such a vector. But it is believed that the concept of the present invention is equally applicable to the construction and utilization of vectors capable of efficient expression of other useful genes encoding, for example, human IFN-$\alpha$, human IFN-$\beta$, $\alpha$-neoendorphin, carcitonin, growth hormones, interleukine 2, and a variety of immunity-associated polypeptides.

1. Amino acid sequences of GIF146 and GIF131 and base sequences coding therefor

The genes GIF146 and GIF131 have the following amino acid sequences.

GIF146:

```
    1                                           10
    Cys Tyr Cys Gln Asp Pro Tyr Val Lys Glu

                                                20
    Ala Glu Asn Leu Lys Lys Tyr Phe Asn Ala

                                                30
    Gly His Ser Asp Val Ala Asp Asn Gly Thr

                                                40
    Leu Phe Leu Gly Ile Leu Lys Asn Trp Lys

                                                50
    Glu Glu Ser Asp Arg Lys Ile Met Gln Ser

                                                60
    Gln Ile Val Ser Phe Tyr Phe Lys Leu Phe

                                                70
    Lys Asn Phe Lys Asp Asp Gln Ser Ile Gln

                                                80
    Lys Ser Val Glu Thr Ile Lys Glu Asp Met

                                                90
    Asn Val Lys Phe Phe Asn Ser Asn Lys Lys

                                                100
    Lys Arg Asp Asp Phe Glu Lys Leu Thr Asn

                                                110
    Tyr Ser Val Thr Asp Leu Asn Val Gln Arg

                                                120
    Lys Ala Ile His Glu Leu Ile Gln Val Met

                                                130
    Ala Glu Leu Ser Pro Ala Ala Lys Thr Gly

                                                140
    Lys Arg Lys Arg Ser Gln Met Leu Phe Arg

                            146
    Gly Arg Arg Ala Ser Gln
```

GIF131:

```
    1                                           10
    Cys Tyr Cys Gln Asp Pro Tyr Val Lys Glu
```

```
                                               20
Ala Glu Asn Leu Lys Lys Tyr Phe Asn Ala
                                               30
Gly His Ser Asp Val Ala Asp Asn Gly Thr
                                               40
Leu Phe Leu Gly Ile Leu Lys Asn Trp Lys
                                               50
Glu Glu Ser Asp Arg Lys Ile Met Gln Ser
                                               60
Gln Ile Val Ser Phe Tyr Phe Lys Leu Phe
                                               70
Lys Asn Phe Lys Asp Asp Gln Ser Ile Gln
                                               80
Lys Ser Val Glu Thr Ile Lys Glu Asp Met
                                               90
Asn Val Lys Phe Phe Asn Ser Asn Lys Lys
                                              100
Lsy Arg Asp Asp Phe Glu Lys Leu Thr Asn
                                              110
Tyr Ser Val Thr Asp Leu Asn Val Gln Arg
                                              120
Lys Ala Ile His Glu Leu Ile Gln Val Met
                                              130
Ala Glu Leu Ser Pro Ala Ala Lys Thr Gly

Lys
```

Chemically synthesized genes that contain the base sequences encoding the above identified amino acid sequences and which are denoted by SUN-GIF146 and SUN-GIF131, respectively, have the following base sequences.

SUN-GIF146:

TGC TAC TGC CAG GAC CCA TAC GTG AAG GAA GCT GAA AAC CTG AAG AAA TAC TTC AAC GCT
ACG ATG ACG GTC CTG GGT ATG CAC TTC CTT CGA CTT TTG GAC TTC TTT ATG AAG TTG CGA

GGT CAT TCT GAC GTT GCT GAC AAC GGT ACT CTG TTC CTG GGT ATC CTG AAA AAC TGG AAA
CCA GTA AGA CTG CAA CGA CTG TTG CCA TGA GAC AAG GAC CCA TAG GAC TTT TTG ACC TTT

GAA GAA TCT GAC CGT AAA ATC ATG CAG TCT CAG ATC GTT TCT TTC TAC TTC AAG CTG TTC
CTT CTT AGA CTG GCA TTT TAG TAC GTC AGA GTC TAG CAA AGA AAG ATG AAG TTC GAC AAG

AAA AAC TTC AAG GAC GAC CAG TCT ATC CAG AAA TCT GTT GAA ACT ATC AAG GAA GAC ATG
TTT TTG AAG TTC CTG CTG GTC AGA TAG GTC TTT AGA CAA CTT TGA TAG TTC CTT CTG TAC

AAC GTT AAG TTC TTC AAC TCT AAC AAG AAA AAG CGT GAC GAC TTC GAA AAG CTT ACT AAC
TTG CAA TTC AAG AAG TTG AGA TTG TTC TTT TTC GCA CTG CTG AAG CTT TTC GAA TGA TTG

TAC TCT GTT ACT GAC CTT AAT GTA CAG CGT AAA GCT ATC CAT GAA CTG ATC CAG GTT ATG
ATG AGA CAA TGA CTG GAA TTA CAT GTC GCA TTT CGA TAG GTA CTT GAC TAG GTC CAA TAC

GCT GAA CTG TCC CCG GCT GCT AAA ACT GGT AAG CGT AAA AGA TCT CAG ATG CTG TTC CGT
CGA CTT GAC AGG GGC CGA CGA TTT TGA CCA TTC GCA TTT TCT AGA GTC TAC GAC AAG GCA

GGT CGT CGT GCT TCT CAG
CCA GCA GCA CGA AGA GTC

EP 0 183 272 B1

S U N - G I F 1 3 1 :

TGC TAC TGC CAG GAC CCA TAC GTG AAG GAA GCT GAA AAC CTG AAG AAA TAC TTC AAC GCT
ACG ATG ACG GTC CTG GGT ATG CAC TTC CTT CGA CTT TTG GAC TTC TTT ATG AAG TTG CGA

GGT CAT TCT GAC GTT GCT GAC AAC GGT ACT CTG TTC CTG GGT ATC CTG AAA AAC TGG AAA
CCA GTA AGA CTG CAA CGA CTG TTG CCA TGA GAC AAG GAC CCA TAG GAC TTT TTG ACC TTT

GAA GAA TCT GAC CGT AAA ATC ATG CAG TCT CAG ATC GTT TCT TTC TAC TTC AAG CTG TTC
CTT CTT AGA CTG GCA TTT TAG TAC GTC AGA GTC TAG CAA AGA AAG ATG AAG TTC GAC AAG

AAA AAC TTC AAG GAC GAC CAG TCT ATC CAG AAA TCT GTT GAA ACT ATC AAG GAA GAC ATG
TTT TTG AAG TTC CTG CTG GTC AGA TAG GTC TTT AGA CAA CTT TGA TAG TTC CTT CTG TAC

AAC GTT AAG TTC TTC AAC TCT AAC AAG AAA AAG CGT GAC GAC TTC GAA AAG CTT ACT AAC
TTG CAA TTC AAG AAG TTG AGA TTG TTC TTT TTC GCA CTG CTG AAG CTT TTC GAA TGA TTG

TAC TCT GTT ACT GAC CTT AAT GTA CAG CGT AAA GCT ATC CAT GAA CTG ATC CAG GTT ATG
ATG AGA CAA TGA CTG GAA TTA CAT GTC GCA TTT CGA TAG GTA CTT GAC TAG GTC CAA TAC

GCT GAA CTG TCC CCG GCT GCT AAA ACT GGT AAG
CGA CTT GAC AGG GGC CGA CGA TTT TGA CCA TTC

2. Construction of improved expression vector for use in GIF146 production

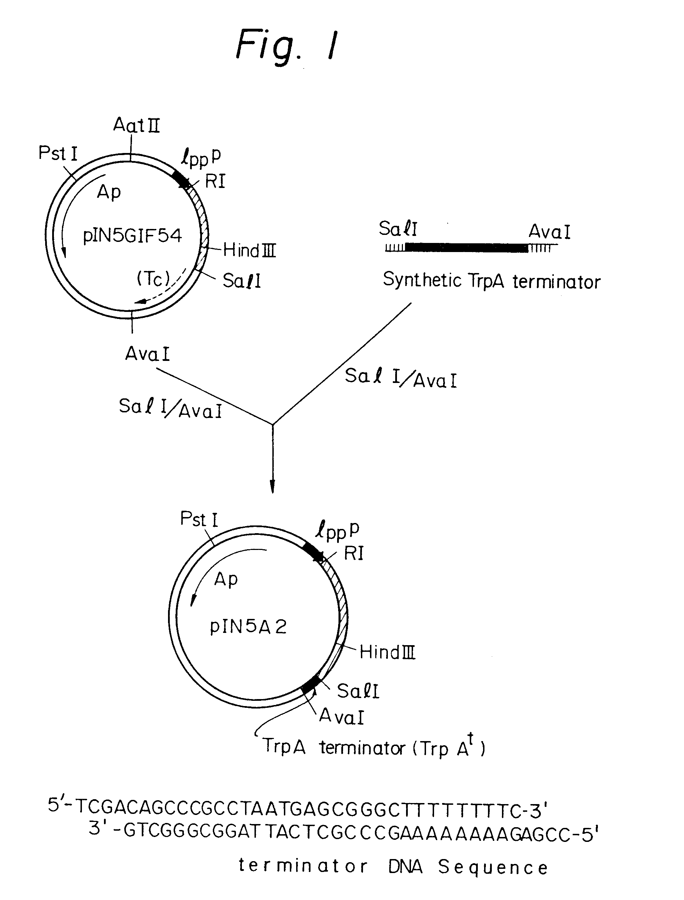

Plasmid pIN5A2 was constructed by the procedures described below. This plasmid was such that, as shown in Fig. 1, a base sequence having the function of terminating the transcription of E. coli TrpA gene (this base sequence is hereunder referred to as a TrpA terminator) was linked immediately downstream from the codon TAA for terminating the translation of a gene encoding GIF146 (see SUN-GIF146) present on the already constructed GIF146 producing plasmid vector pIN5GIF54 (disclosed in Japanese Patent Application No. 132524/1983).

It has been reported that the TrpA terminator is an efficient $\rho$-factor independent terminator (Christie, G.E. et al., Proc. Natl. Acad. Sci., USA, 78, 4180, 1981). A DNA fragment of this terminator is commercially available from Pharmacia Japan Co., Ltd. (Tokyo). The present inventors obtained a sample of this DNA fragment and added the sticky end of SalI to one terminus of the base sequence, and the sticky end of AvaI to the other terminus The resultant DNA fragment had the following base sequence:

```
    SalI sticky end                         AvaI sticy end
      5'-TCGACAGCCCGCCTAATGAGCGGGCTTTTTTTTC-3'
         GTCGGGCGGATTACTCGCCCGAAAAAAAAGAGCC
```

This fragment was used as a TrpA terminator. The plasmid pIN5GIF54 was a GIF146 producing plasmid having a lipoprotein promoter region (lppP) including an improved Shine-Dargarno sequence; in this plasmid, the terminator of tetracycline resistance gene (Tc$^r$) was present downstream from the codon for terminating the translation of GIF146 coding gene.

The present inventors cut pIN5GIF54 with SalI and AvaI and inserted into the cleavage site the TrpA terminator having SalI and AvaI sticky termini at the two ends, thereby constructing plasmid pIN5A2 having the TrpA terminator linked immediately downstream from the translation termination codon (see Fig. 1). Subsequently, E. coli W3110 was routinely transformed by this plasmid. Another strain of E. coli W3110 was transformed by unmodified pIN5GIF54. The two transformants were cultured in a GC medium (3% glycerin, 2% Casamino acids, 0.2% yeast extract, 0.1% MgSO$_4$·7H$_2$O and 0.5% KH$_2$PO$_4$, pH, 6.5) at 36°C for 24 hours. After cultivation, the activity of interferon and the amount of GIF146 produced were determined. The interferon activity was measured in terms of the anti-viral activity of the extract obtained by freezing and thawing the lysozyme-treated cultured cells. The amount of GIF146 production was measured by the following procedures: the cells were heat-treated with SDS to obtain the total protein, which was subjected to SDS-polyacrylamide gel electrophoresis (hereunder abbreviated as SDS-PAGE), stained with Coomassie blue, and subjected to gel scanning at 580 nm for calculating the proportion of ca. 18 kd protein band to the total protein; with the strain W3110 bearing no GIF146 expression vector, the 18 kd protein band was observed in an amount of about 3% of the total protein, and this portion of the 18 kd band was regarded as the background and counted out from the calculation of the quantity of GIF146 relative to the total protein content.

As shown in Table 1, E. coli W3110/pIN5A2 transformed by the plasmid pIN5A2 having the TrpA terminator linked immediately downstream from the translation termination codon had an anti-viral activity about twice the value for E. coli W3110/pIN5GIF54 transformed by unmodified pIN5GIF54, and the amount of GIF146 produced by the former strain was about 1.7 times as large as the value for the latter. This obviously shows the meritorious effects achieved by inserting the TrpA terminator immediately downstream from the termination codon.

Table 1

| Comparison of $\gamma$-IFN productivity | | | |
|---|---|---|---|
| Strain | Antiviral activity* | % of 18 kd protein | % of GIF |
| W3110/pIN5GIF54 | $3.0 \times 10^4$ | 12% | 9% |
| W3110/pIN5A2 | $6.1 \times 10^4$ | 18% | 15% |
| W3110 | 0 | 3% | 0% |

* unit/ml culture

Plasmid pIN5T4 containing no TrpA terminator was constructed from pIN5GIF54 and pBR322. At the same time, plasmid pIN5T8 having the TrpA terminator linked immediately downstream from the codon for terminating the translation of the GIF146 structural gene was constructed from pIN5A2 (see Fig. 1) and pBR322. The two plasmids, pIN5T4 and pIN5T8, differed only with respect to the presence of TrpA terminator, and both had tetracycline resistance (Tc$^r$) rather than ampicillin resistance (Ap$^r$) as the drug resistance marker on the plasmid shows in Fig. 1. Two strains of E. coli W3110 were respectively transformed by the two plasmids, and the anti-viral activity of each transformant and the amount of its GIF146 production were measured by the procedures described above. The results are shown in Table 2, from which one can see that E. coli W3110/pIN5T8 transformed by pIN5T8 exhibited an antiviral activity about 4 times as high as the value for E. coli W3110/pIN5T4 transformed by the TrpA terminator free pIN5T4, and the amount of GIF produced by the former E. coli strain was about 3 times as large as the value achieved by the latter strain. These data obviously show the meritorious effects of inserting the TrpA terminator immediately downstream from the translation termination codon.

Table 2

| Comparison of γ-IFN productivity | | | |
|---|---|---|---|
| Strain | Antiviral activity* | % of 18 kd protein | % of GIF |
| W3110/pIN5T4 | $7.6 \times 10^3$ | 14% | 11% |
| W3110/pIN5T8 | $3.0 \times 10^4$ | 35% | 32% |
| W3110 | 0 | 3% | 0% |

* unit/ml culture

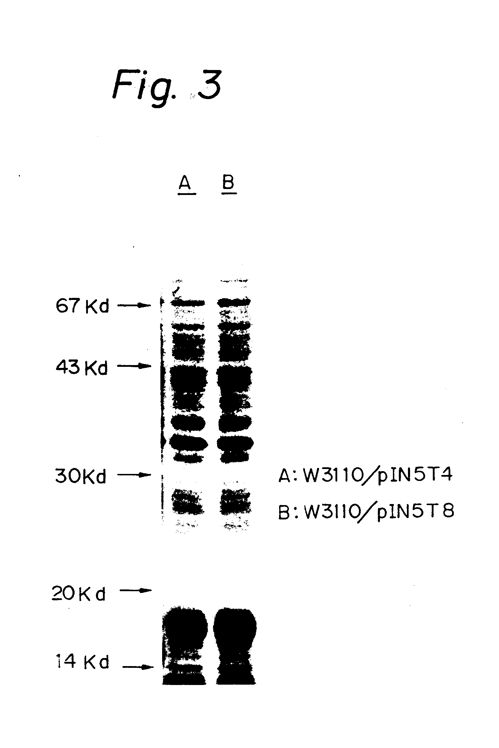

A photomicrograph of the protein bands obtained by SDS-PAGE and strained with Coomassie blue is shown in Fig. 3.

The present inventors continued their experimentation using plasmids having structures that differed slightly from those described above. As a result, the meritorious effects of inserting the TrpA terminator were verified, and at the same time, it was demonstrated that linking the TrpA terminator immediately downstream from the codon for the termination of the translation of the structural gene to be expressed was essential for the present invention.

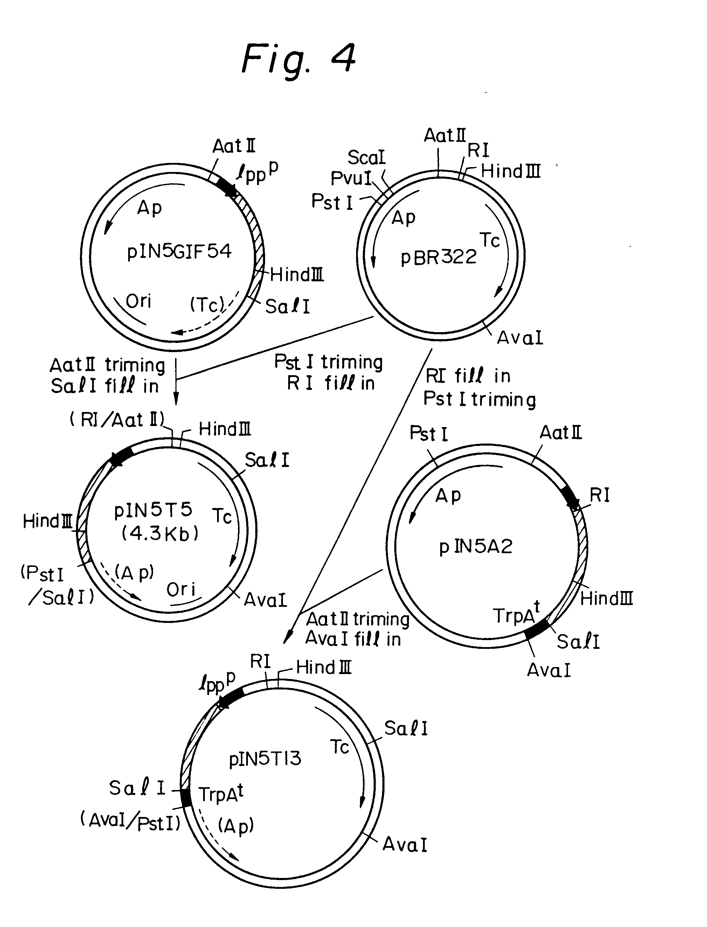

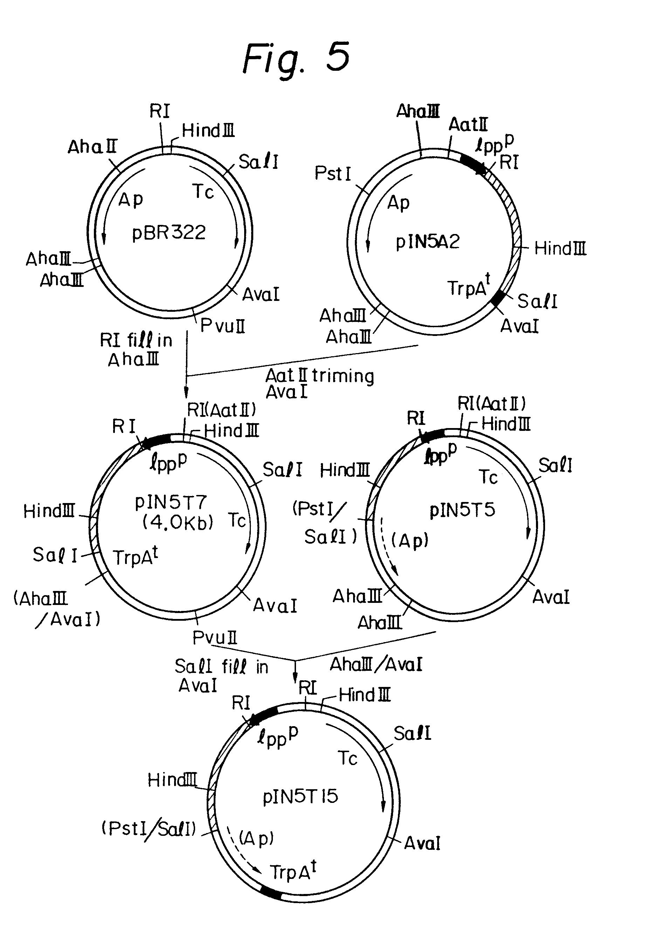

Three plasmids were constructed as shown below. The first one was plasmid pIN5T5 where that region of the ampicillin-resistance gene (Ap$^r$) on pBR322 which was downstream from the PstI cleavage site (in the Ap$^r$ structural gene) was linked immediately downstream from the translation termination codon of the GIF146 structural gene. The second was plasmid pIN5T13 where the TrpA terminator was linked immediately downstream from the same termination codon, and immediately downstream from said terminator was linked that region of Ap$^r$ which was downstream from the PstI cleavage site (see Fig. 4). The third one was plasmid pIN5T15 which was constructed by the procedures shown in Fig. 5 and wherein that region of Ap$^r$ which was downstream from the PstI site was inserted between the termination codon of the GIF146 structural gene and the TrpA terminator. In pIN5T15, the TrpA terminator was inserted 370 bp downstream from the termination codon of the GIF146 structural gene.

Three strains of E. coli W3110 were transformed by the plasmids, pIN5T5, pIN5T13 and pIN5T15, respectively, and the resulting transformants E. coli W3110/pIN5T5, W3110/pIN5T13 and W3110/pIN5T15 were examined for their antiviral activity and GIF146 production. Untransformed host E. coli W3110 was used as the control. The results are shown in Table 3.

Table 3

| Comparison of γ-IFN productivity | | | |
|---|---|---|---|
| Strain | Antiviral activity* | % of 18 kd protein | % of GIF |
| W3110/pIN5T5 | $3.0 \times 10^4$ | 22% | 19% |
| W3110/pIN5T13 | $6.1 \times 10^4$ | 36% | 33% |
| W3110/pIN5T15 | $3.0 \times 10^4$ | 16% | 13% |
| W3110 | 0 | 3% | 0% |

* unit/ml culture

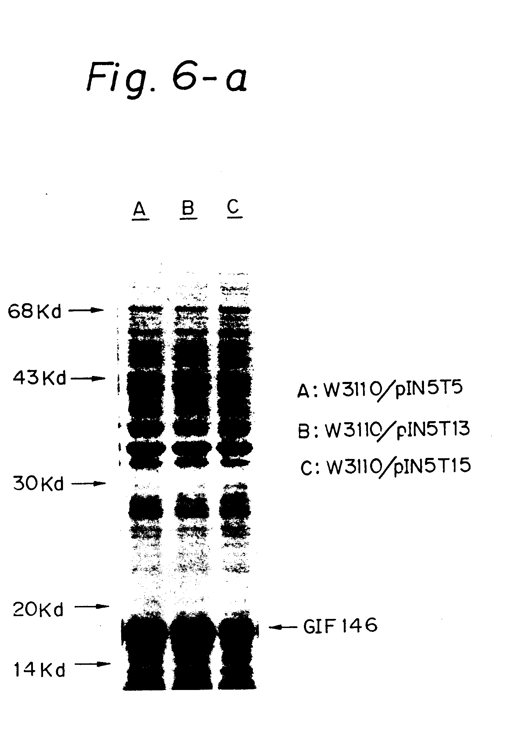

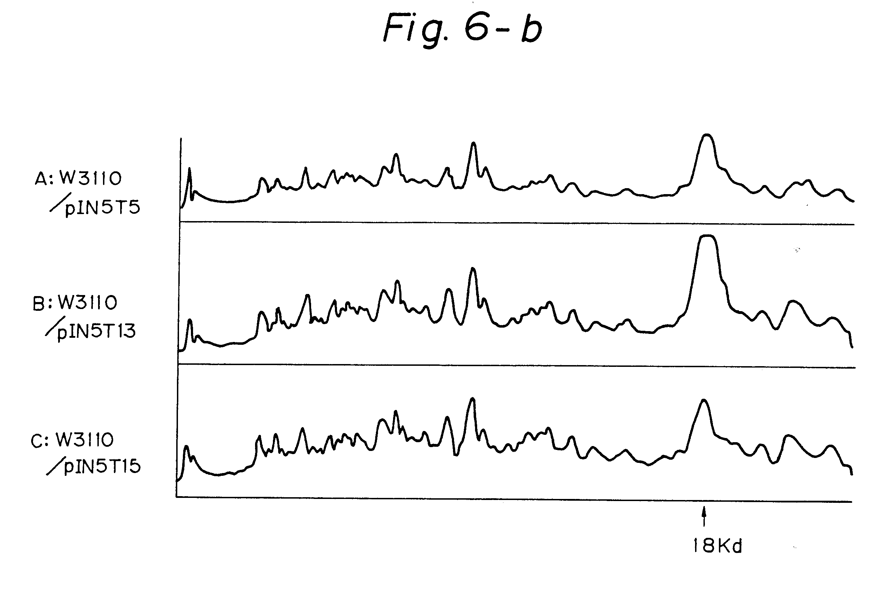

Fig. 6-a is a photomicrograph of protein bands obtained by SDS-PAGE and stained with Coomassie blue, and Fig. 6-b shows protein patterns obtained by gel-scanning these bands. The band for ca. 18 kb corresponds to GIF146 protein.

The data in Table 3 and Fig. 6 show the following: E. coli W3110/pIN5T13 transformed by plasmid pIN5T13 when the TrpA terminator was linked immediately downstream from the termination codon of the GIF146 structural gene exhibited an anti-viral activity and GIF146 productivity that were about twice and 1.7 times, respectively, as high as the values for E. coli W3110/pIN5T5 and W3110/pIN5T15 which were respectively transformed by plasmid pIN5T5 having no TrpA terminator insert and plasmid pIN5T15 where the TrpA terminator was present at a distance from the termination codon. It is therefore clear that the TrpA terminator linked immediately downstream from the termination codon was effective for the purpose of enhancing the productivity of the exogenous protein GIF146.

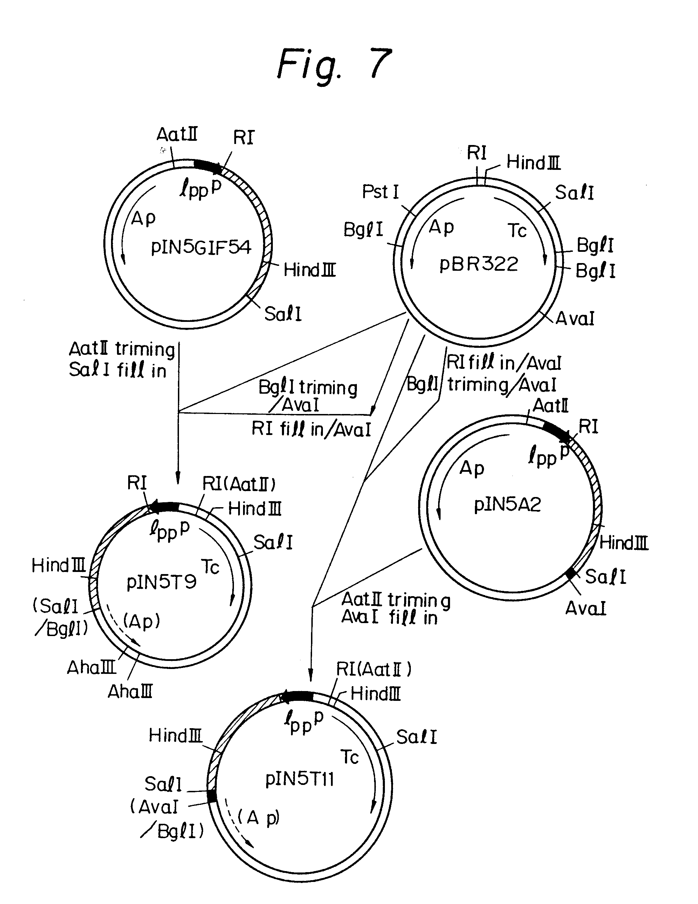

The present inventors constructed two additional plasmids, pIN5T11 and pIN5T9, by the procedures shown in Fig. 7. The plasmid pIN5T11 was such that the TrpA terminator was linked immediately downstream from the termination codon of the GIF146 structural gene, and pIN5T9 was the plasmid harboring no TrpA terminator. These plasmids were introduced into two strains of E. coli W3110 and the transformants were examined for their ability to produce GIF146. The results are shown in Table 4, from which one can see that E. coli W3110/pIN5T11 transformed by plasmid pIN5T11 where the TrpA terminator was linked immediately downstream from the termination codon of the GIF146 structural gene achieved GIF146 production about twice the value for E. coli W3110/pIN5T9 transformed by plasmid pIN5T9 having no TrpA terminator insert. These data again demonstrate the meritorious effects of inserting the TrpA terminator.

Table 4

| Comparison of $\gamma$-IFN productivity | | | |
|---|---|---|---|
| Strain | Antiviral activity[*] | % of 18 kd protein | % of GIF |
| W3110/pIN5T9 | $3.0 \times 10^4$ | 20% | 17% |
| W3110/pIN5T11 | $6.1 \times 10^4$ | 36% | 33% |
| W3110 | 0 | 3% | 0% |

[*] unit/ml culture

Both pIN5T9 (Fig. 7) and pIN5T5 were plasmids harboring no TrpA terminator. In pIN5T5, that region of Ap[r] on pBR322 which was downstream from the PstI cleavage site was linked downstream from the termination codon of the GIF146 structural gene, and in pIN5T9, that region of Ap[r] which was downstream from the BglI cleavage site 127 bp downstream from the PstI site was linked downstream from the same termination codon. As can be seen from Tables 3 and 4, E. coli W3110/pIN5T5 and W3110/pIN5T9 transformed respectively by pIN5T5 and pIN5T9 were substantially equal to each other with respect to the productivity of GIF146 and antiviral activity.

3. Linking TrpA terminator to GIF131 producing vector

The present inventors also checked the effects of linking the TrpA terminator downstream from the translation termination codon in a vector for production of GIF131 where 15 amino acid residues were deleted from the C terminus of GIF146.

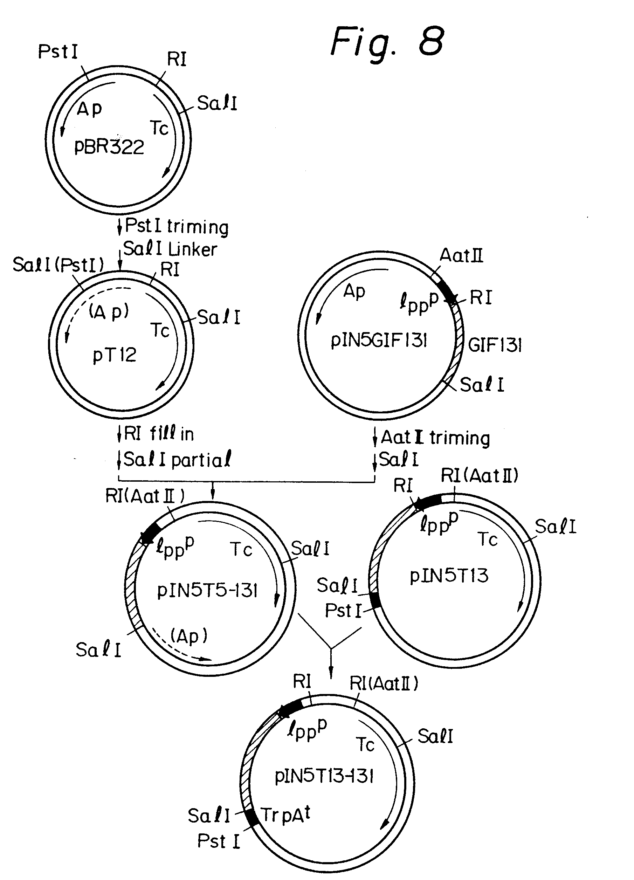

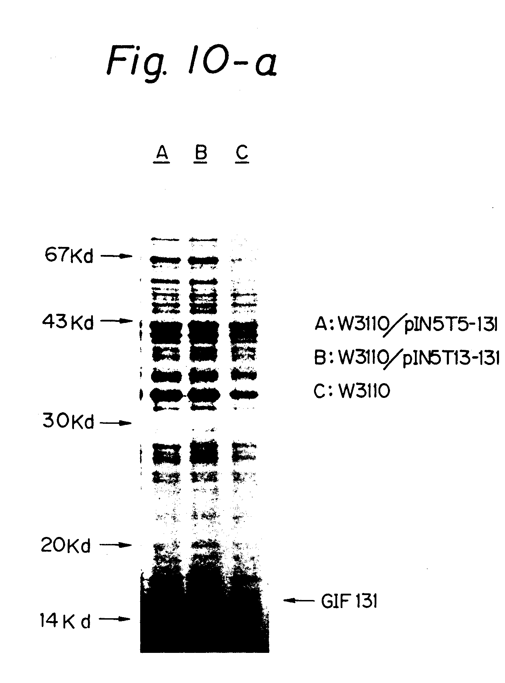

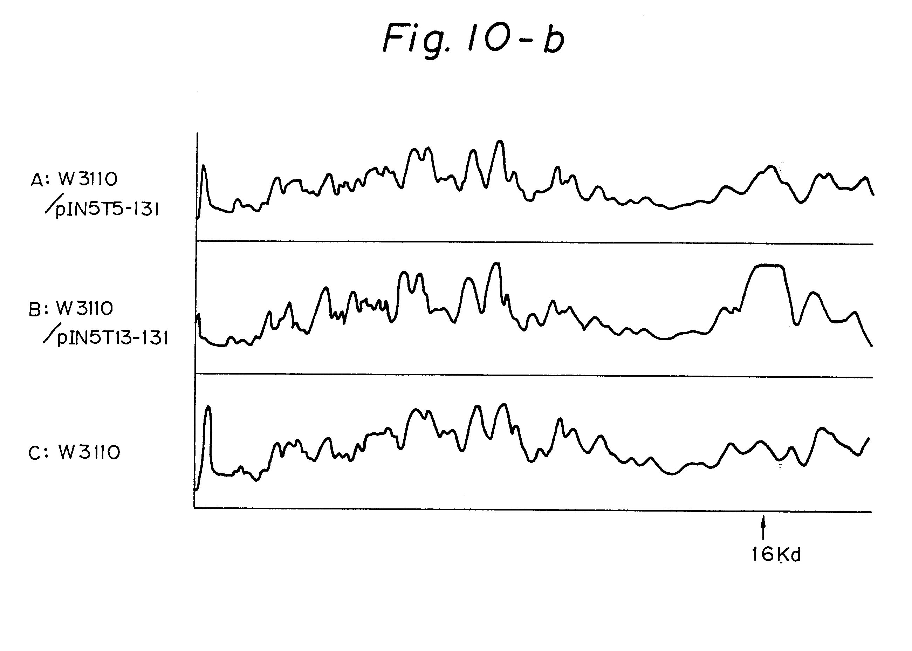

By the procedures illustrated in Fig. 8, two plasmids, pIN5T5-131 and pIN5T13-131, were constructed, wherein the GIF146 structural gene portions present on pIN5T5 and pIN5T13 (see Fig. 4) were replaced by the corresponding GIF131 structural gene portions. These plasmids were introduced into two strains of E. coli W3110 and the resultant transformants were checked for their GIF131 productivity and antiviral activity. In pIN5T13-131, the TrpA terminator was linked immediately downstream from the translation termination codon of the GIF131 structural gene, and in pIN5T5-131, no such terminator was inserted. Fig. 10-a shows the protein bands obtained by SDS-PAGE of fractions extracted from the cultured cells of the two transformants and the control W3110 strain, and Fig. 10-b depicts the patterns obtained by gel-scanning these protein bands. The band or peak for ca. 16 kd corresponds to the GIF131 protein. As is clear from Fig. 10, particularly from Fig. 10-b, E. coli W3110/pIN5T13-131 transformed by pIN5T13-131 where the TrpA terminator was linked immediately downstream from the termination codon of GIF131 structural gene achieved more efficient production of GIF131 than E. coli W3110/pIN5T5-131 transformed by pIN5T5-131 having no such terminator. As shown in Table 5 below, W3110/pIN5T13-131 exhibited an antiviral activity

11

and GIF131 productivity which were respectively at least 4 times and about 2.5 times as high as the values for W3110/pIN5T5-131. It was therefore confirmed that "linking a terminator immediately downstream from the translation termination codon" in accordance with the present invention is effective for enhancing the production not only of GIF146 but also of another exogeneous protein, or GIF131 in the specific embodiment described above.

The plasmid pIN5GIF131 shown in Fig. 8 was so constructed as to ensure efficient production of GIF131 under the control of an improved promoter region of lipoprotein (lpp) gene. For details of the method of constructing this plasmid, see Japanese Patent Application No. 241457/1983.

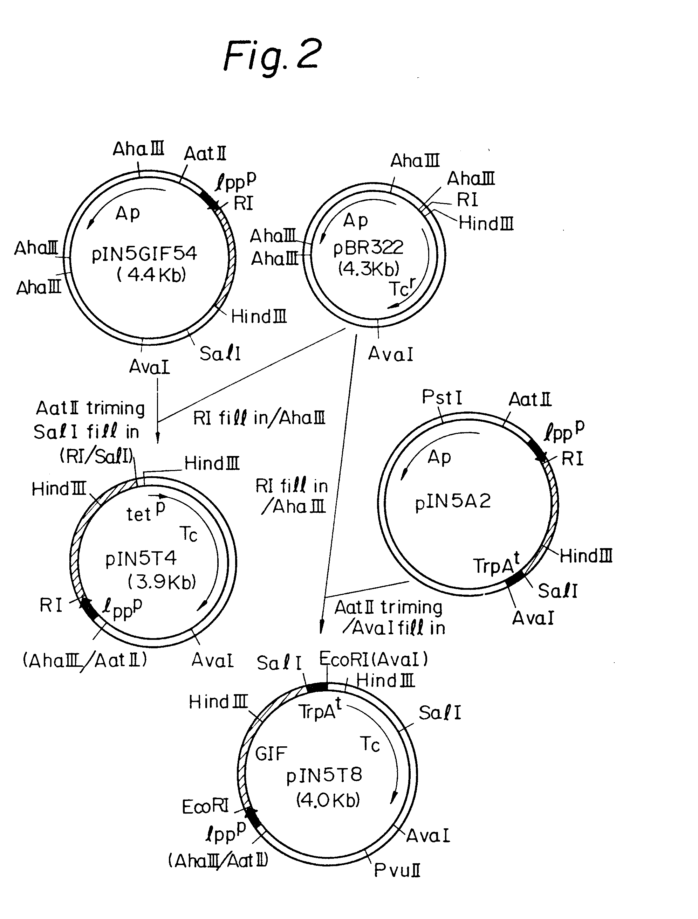

In order to further verify the effects of "terminator insertion" in GIF131 production, the present inventors constructed two more plasmids, pIN5T11-131 and pIN5T8-131, wherein the GIF146 structural gene portions on pIN5T11 (see Fig. 7) and pIN5T8 (see Fig. 2), respectively, were replaced by GIF131 structural genes. After introducing these plasmids into two strains of E. coli W3110, the transformants were examined for their GIF131 productivity and antiviral activity. Each of these plasmids was such that the TrpA terminator was linked immediately downstream from the termination codon of the GIF131 structural gene. The GIF131 productivity and antiviral activity values for the two transformants are shown in Table 5, and they were identical to each other with respect to those factors. In comparison with E. coli W3110/pIN5T5-131 transformed by pIN5T5-131 having no "insertion of a terminator", the GIF131 productivity and antiviral activity values for these two transformants were about 3- and 4-fold, respectively. Table 5 therefore demonstrates the meritorious effects of "inserting a terminator".

Table 5

| Comparison of GIF131 productivity | | | |
|---|---|---|---|
| Strain | Antiviral activity* | % of 16 kd protein | % of GIF-131 |
| W3110/pIN5T5-131 | $1.5 \times 10^4$ | 9% | 6% |
| W3110/pIN5T13-131 | $6.1 \times 10^4$ | 18% | 15% |
| W3110/pIN5T11-131 | $6.1 \times 10^4$ | 21% | 18% |
| W3110/pIN5T8-131 | $6.1 \times 10^4$ | 21% | 18% |
| W3110 | 0 | 3% | 0% |
| * unit/ml culture | | | |

As will be understood from the foregoing description, the "insertion of a terminator" in accordance with the present invention is very effective for the purpose of ensuring efficient production of an exogenous protein or polypeptide in a host microorganicsm, particularly E. coli. Obviously, the present invention will offer great industrial benefits by realizing efficient production not only of the proteins mentioned hereinabove and in the following example (GIF146 and GIF131) but also of other proteins and polypeptides.

The present invention will be described in greater detail with reference to the following example.

Example

Plasmid vector capable of efficient gene expression by virtue of insertion of a terminator:

1. Preparation of pIN5A2

Three micrograms of pIN5GIF54 (see Japanese Patent Application No. 132524/1983) was completely digested with 15 units of SalI and 15 units of AvaI. The digested plasmid was subjected to agar-gel electrophoresis and the larger DNA fragment was electro-eluted from the gel by electrophoresis. DNA recovery was subsequently effected by precipitation with ethanol. A TrpA transcription terminator was designed (for its base sequence, see Table 1) so that it had cohesive SalI and AvaI (AGCC) termini at the ends. This DNA fragment was synthesized by a DNA synthesizer, Model 380 A of Applied Biosystems, and elimination of protective groups and purification were performed by routine techniques. The purified DNA fragment was phosphated at 5' terminus with ATP and polynucleotide kinase. A portion (0.2 $\mu$g) of the phosphated DNA fragment was mixed with the previously obtained SalI - AvaI fragment of pIN5GIF54 and the mixture was subjected to ligation at 15°C for 18 hours in a ligation solution (20 $\mu$l) composed of 20 mM tris-HCl (pH, 7.4), 10 mM MgCl$_2$, 10 mM DTT (dithiothreitol), and 1 mM ATP. After treatment with 0.3 mL CaCl$_2$, E. coli W3110 was transformed by addition of 10 $\mu$l of the ligation solution. Ampicillin-resistant clones of the transformant were analyzed routinely to obtain W3110 clones having pIN5A2 (see Fig. 1). The productivity of IFN[7]-γ (GIF146) using the transformant with pIN5A2 and pIN5GIF54 was checked by the following procedures.

The transformant was shake-cultured at 30°C for 24 hours in 1.5 ml of a GC medium in a 16.5 mm test tube; the medium contained 40 $\mu$g/ml of ampicillin and had the following composition: 2% glycerol, 3% Casamino acids, 0.2% yeast extract, 0.5% $KH_2PO_4$ and 0.1% $MgSO_4 \cdot 7H_2O$, and pH adjusted to 6.5 with NaOH. After completion of the cultivation ($OD_{660} \simeq 7$), 0.5 ml of the culture solution was put into a 1.5 ml Eppendorf cup and centrifuged at 10,000 rpm for 5 minutes. The collected cells were suspended in 0.5 ml of PBS solution (0.8% NaCl, 0.02% KCl, 0.115% sodium monohydrogenphosphate, and 0.02% potassium dihydrogenphosphate) containing 1 mg/ml of lysozyme and 1 mM EDTA. After reaction at 0°C for 30 minutes, the cells were disrupted by three freeze-thaw cycles. The supernatant fraction was recovered by centrifugation at 10,000 rpm for 10 minutes and checked for antiviral activity by the method shown in Unexamined Published Japanese Patent Application No. 201995/1983.

Another 0.5-ml portion of the culture solution was centrifuged for 5 minutes at 10,000 rpm. The collected cells were dissolved in 200 $\mu$l of SDS sample solution (10 mM phosphate buffer with pH 7.2, 7 M urea, 1% SDS, and 1% 2-mercaptoethanol) and the resulting solution was heated on a boiling water bath for 10 minutes. A portion (20 $\mu$l) of the sample was separated into fractions by 13% SDS-PAGE and the protein was stained with Coomassie blue. The protein bands were subjected to gel-scanning at 580 nm to calculate the proportion of GIF146 to the total protein. GIF146 produced a band at ca. 18 kd, but the untransformed W3110 would produce the same band in an amount of ca. 3%, so this value was subtracted from the observed value of GIF146 protein relative to the total protein. The results of the measurements of antiviral activity and GIF146 production are shown in Table 1, from which one can see that pIN5A2 having the TrpA terminator inserted immediately downstream from the GIF146 gene had antiviral activity and GIF146 productivity values which are respectively about 2 and 1.7 times as high as the values exhibited by pIN5GIF54.

## 2. Preparation of pIN5T4 and pIN5T8

A portion (5 $\mu$g) of pIN5GIF54 was completely digested with 20 units of AatII and 20 units of SalI. The cohesive AatII end (3' terminus) and SalI end (5' terminus) were made blunt using T4 DNA polymerase and 4dNTP (including dATP, dGTP, dCTP and TTP); the AatII end was subjected to trimming whereas the SalI end was subjected to filling-in. The digested plasmid was separated by agar electrophoresis and a DNA fragment (ca. 750 bp) containing the lipoprotein promoter (lppP) and GIF146 gene was obtained by the method described above.

A portion (5 $\mu$g) of pBR322 was completely digested with 20 units of EcoRI and the resulting sticky end was made blunt by the filling-in technique described above. The plasmid was then digested completely with 20 units of AhaIII. The digested plasmid was separated by agar electrophoresis and a DNA fragment (ca. 3.3 kd) containing the DNA replication initiation point and tetracycline-resistance gene was obtained by the method described above.

The two DNA fragments were mixed, ligated and treated by the method described above to obtain pIN5T4 (see Fig. 2).

A portion (5 $\mu$g) of pIN5A2 was completely digested with 20 units of AatII and 20 units of AvaI, and the resulting cohesive ends were made blunt by the trimming and filling-in techniques described above. The digested plasmid was separated by agar electrophoresis and a DNA fragment (ca. 800 bp) containing the GIF146 gene and TrpA terminator was obtained by the method described above. This DNA fragment was mixed with the pBR322-derived DNA fragment (ca. 3.3 kb) described hereinbefore, and the mixture was ligated and treated by the procedures described above to obtain pIN5T8 (Fig. 2).

Two strains of E. coli W3110 were transformed by pIN5T4 and pIN5T8, respectively, and the antiviral activity and GIF146 productivity of each of the transformants were determined by the same methods as described above. The results are shown in Tables 2 and 3; pIN5T8 exhibited an antiviral activity about 4 times as high as the value achieved by pIN5T4, and the former was about 3 times more efficient in GIF146 production than the latter (i.e., 32% of the total protein). Fig. 3 shows a photomicrograph of the protein bands obtained by SDS-PAGE and strained with Coomassie blue, and the efficiency of expression by pIN5T8 as compared with pIN5T4 is also apparent from this photomicrograph. The above data will demonstrate the meritorious effects of inserting a terminator.

## 3. Preparation of pIN5T5, pIN5T13 and pIN5T15

Two plasmids, pIN5T5 and pIN5T9 (to be described later), were first prepared. By inserting the TrpA terminator into pIN5T5, pIN5T13 and pIN5T15 were constructed, and by inserting the same terminator into pIN5T9, pIN5T11 (to be described later) was constructed.

Starting from pIN5GIF54, a blunt-ended AatII - SalI DNA fragment (ca. 750 bp) containing lppP (lpp promoter) and GIF gene was obtained by the method described in 2. A portion (5 $\mu$g) of pBR322 was completely digested with 20 units of PstI and 20 units of EcoRI and the resulting cohesive ends were made blunt by the method described above, so as to obtain a 3.6 kb DNA fragment containing the replication initiation point, tetracycline-resistance gene and that portion of the $\beta$-lactamse gene which was downstream from the PstI site. The two DNA fragments were mixed together and ligated to construct pIN5T5 (see Fig. 4).

The pBR322-derived PstI - EcoRI DNA fragment (3.6 kb) with flush ends was mixed with a pIN5A2-derived flush-ended AatII - AvaI (0.8 kb) DNA fragment obtained by the same method as described in 2, and the mixture was ligated and treated by the method described above to obtain pIN5T13.

By employing the following procedures, pIN5T15 having the TrpA terminator inserted ca. 380 bp downstream from the GIF146 gene was constructed (Fig. 5). A portion (5 $\mu$g) of pBR322 was cut with 20 units of EcoRI and the resulting cohesive end was filled in with T4 DNA polymerase using the method described above. Thereafter, the plasmid was cut with 20 units of AhaIII and treated by the method described above to obtain a DNA fragment (3.2 kb) containing the tetracycline-resistance gene. This DNA fragment was mixed with a pIN5A2-derived flush-ended AatII - AvaI (0.8 kb) DNA fragment that was obtained from 5 $\mu$g of digested pIN5A2 by the method described above. The mixture was ligated and treated by the method described above to obtain pIN5T7. A portion (5 $\mu$g) of pIN5T7 was digested with 30 units of SalI and the resulting cohesive end was filled in by the same method as described above. The plasmid was then digested with 20 units of AvaI and treated by the method described above to obtain a DNA fragment (1.8 kg) containing the replication initiating point. A portion (5 $\mu$g) of pIN5T5 was completely digested with 20 units of AhaIII and 20 units of AvaI and subsequently treated by the method described above to obtain a DNA fragment containing the GIF146 gene and tetracycline-resistance gene. The two DNA fragments were mixed, ligated and subsequently treated to obtain pIN5T15.

Three strains of E. coli W3110 were transformed by pIN5T5, pIN5T13 and pIN5T15, respectively, and the resulting transformants were cultured and analyzed by the procedures described in 2. Fig. 6 shows a photomicrograph of the protein bands obtained by SDS-PAGE and stained with Coomassie blue, and the protein patterns obtained by gel-scanning the bands. The plasmid pIN5T13 having the TrpA terminator inserted immediately downstream from the GIF146 gene was more efficient than pIN5T5 in production of the 18 kd GIF146 protein. Table 3 summarizes data for the antiviral activity and the proportion of GIF146 protein to total protein as determined by gel-scanning analysis. The plasmid pIN5T13 capable of efficient gene expression had an antiviral activity twice as high as the value achieved by pIN5T5, and produced the GIF146 protein in an amount 1.7 times as large as that produced by pIN5T5. The third plasmid pIN5T15 having the TrpA terminator inserted 380 bp downstream from the structural gene was comparable to pIN5T5 in the efficiency of gene expression. These results show that the insertion of the TrpA terminator is effective only when it is located immediately downstream of the structural gene.

4. Preparation of pIN5T9 and pIN5T11

In pIN5T5, the 3' terminal end of GIF146 gene was located at the PstI site of $\beta$-lactamase gene on pBR322. In this section, the results of experiment with a plasmid where the 3' terminal end of GIF146 gene was located at the BglI site of $\beta$-lactamse gene are described.

Fig. 7 shows the procedures for preparing pIN5T9 and pIN5T11 (having the TrpA terminator inserted immediately downstream from the GIF146 gene in pIN5T9). A portion (5 $\mu$g) of pIN5GIF54 was digested with 20 units of AatII and 20 units of SalI and the resulting cohesive ends were made blunt by the method described above. Thereafter, a DNA fragment containing the GIF146 gene was separated. A portion (5 $\mu$g) of pBR322 was cut with 20 units of BglI and the resulting sticky end was made blunt. The plasmid was then cut with 20 units of AvaI and treated by the method described above to obtain a DNA fragment containing the replication initiation point. A portion (5 $\mu$g) of pBR322 was cut with 20 units of EcoRI, made blunt-ended, cut with 20 units of AvaI, and treated by the method described above to obtain a DNA fragment containing the tetracycline-resistance gene. The three DNA fragments were mixed together and ligated to obtain pIN5T9.

A portion (5 $\mu$g) of pIN5A2 was digested with 20 units of AatII and 20 units of AvaI and treated by the method described above to obtain a flush-ended DNA fragment containing the GIF146 gene. A portion (5 $\mu$g) of pBR322 was cut with 20 units of BglI, made flush-ended, cut with 20 units of AvaI, and treated by the

EP 0 183 272 B1

method described above to obtain a DNA fragment containing the replication initiation point. A portion (5 μg) of pBR322 was cut with 20 units of EcoRI, made blunt-ended, cut with 20 units of AvaI, and treated by the method described above to obtain a DNA fragment containing the tetracycline-resistance gene. The three DNA fragments were mixed together and ligated to obtain pIN5T11. Two strains of E. coli W3110 were transformed by pIN5T9 and pIN5T11, respectively, and the resulting transformants were analyzed for their GIF146 productivity by the method described in 2. The results are shown in Table 4, from which one can see that pIN5T11 having the TrpA terminator inserted immediately downstream from the GIF146 gene was about twice as efficient as the terminator-free pIN5T9 with regard to both antiviral activity and GIF146 production.

5. Preparation of pIN5T5-131, pIN5T13-131, pIN5T8-131 and pIN5T11-131

In this section are reviewed the effects of terminator insertion on the expression of GIF131 gene (as described in Japanese Patent Application No. 241457/1983, where 15 amino acid residues were deleted from C-terminus).

Fig. 8 shows the sequence of steps for preparing pIN5T5-131 and pIN5T13-131. A portion (5 μg) of pBR322 was cut with 10 units of PstI, made blunt-ended by the method described above, and ligated with 0.5 μg of SalI linker, whereby pT12 having SalI inserted into the PstI cleavage site was obtained. A portion (5 μg) of pT12 was completely digested with 20 units of EcoRI, made blunt-ended by the method described above, partially digested with 1 unit of SalI and subsequently treated by the method described above to obtain a DNA fragment containing the tetracycline resistance gene.

A portion (5 μg) of pIN5GIF131 (described in Japanese Patent Application No. 241457/1983) was digested with 20 units of AatII, made blunt-ended by the method described above, digested with 20 units of SalI and subsequently treated by the method described above to obtain a DNA fragment containing the GIF131 gene.

The two DNA fragments were mixed together and ligated to obtain pIN5T5-131.

A portion (5 μg) of the pIN5T5-131 was digested with 40 units of SalI and subsequently treated by the method described so as to obtain a DNA fragment containing the GIF131 gene and part of the tetracycline-resistance gene. A portion (5 μg) of pIN5T13 (see Fig. 4) was digested with 40 units of SalI and subsequently treated by the method described above to obtain a DNA fragment containing part of the tetracycline-resistance gene, the TrpA terminator and the replication initiation point. The two DNA fragments were mixed and ligated to obtain pIN5T13-131. This plasmid was identical to pIN5T5-131 except that the TrpA terminator was inserted immediately downstream from the GIF131 gene.

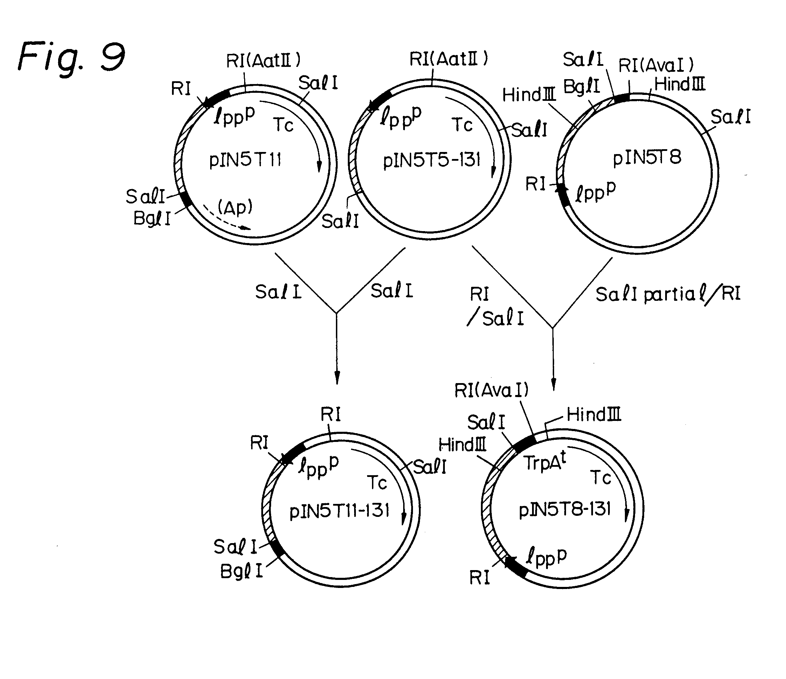

Fig. 9 shows the sequence of steps for preparing pIN5T8-131 and pIN5T11-131, both of which had the TrpA terminator inserted immediately downstream from the GIF131 gene. Two plasmids, pIN5T11 (see Fig. 7) and pIN5T5-131, each weighing 5 μg were digested with 40 units of SalI. From the pIN5T11, a DNA fragment containing part of the tetracycline-resistance gene, the TrpA terminator and the replication initiation point was obtained by the method described above. From the pIN5T5-131, a DNA fragment containing the GIF131 gene and part of the tetracycline-resistance gene was obtained by the method described above. These two DNA fragements were mixed and ligated to obtain pIN5T11-131.

A portion (5 μg) of pIN5T5-131 was digested with 20 units of EcoRI and 20 units of SalI, and subsequently treated by the method described above to obtain a DNA fragment containing the GIF131 gene. A portion (5 μg) of pIN5T8 was completely digested with 20 units of EcoRI, partially digested with 1 unit of SalI, and subsequently treated by the method described above to obtain a DNA fragment containing the tetracycline-resistance gene, TrpA terminator, lpp promoter and the replication initiation point. The two DNA fragments were mixed and ligated to obtain pIN5T8-131.

Strains of E. coli W3110 were transformed by the plasmids constructed above and the efficiency of their production of GIF131 was investigated by the method described above. Fig. 10 shows the results of analysis of the SDS-PAGE bands for pIN5T5-131 and pIN5T13-131 (as obtained by protein staining and gel-scanning).

These results show that pIN5T13-131 having the TrpA terminator inserted immediately downstream from the GIF131 to be expressed had a higher GIF131 productivity than pIN5T5-131 having no TrpA terminator insert. Table 5 lists the antiviral activity values for the GIF131 producing plasmids and the results of gel-scanning of the protein bands obtained by SDS-PAGE and stained with Coomassie blue. The plasmid pIN5T13-131 obviously exhibited the effects of terminator insertion because its antiviral activity and GIF131 productivity were 4 and 2.5 times as high as the values for pIN5T5-131, respectively. Results similar to those obtained by pIN5T13-131 were attained by pIN5T8-131 where the expression unit (i.e., the DNA fragment having the lpp promoter, GIF131 gene and the TrpA terminator linked together) was inserted in the

15

opposite direction as compared with pIN5T13-131, and by pIN5T11-131 wherein the same expression unit was inserted in a position different from that adopted in pIN5T13-131.

**Claims**

**Claims for the following Contracting States : BE, CH, DE, FR, GB, IT, LI, LU, NL, SE**

1. A vector capable of efficient expression of a gene encoding a protein or polypeptide with an interferon activity or anti-tumor activity, characterized in that the E. coli trpA terminator is linked immediately downstream from the stop codon of said gene to be expressed.

2. A vector according to Claim 1 wherein the protein or polypeptide has a gamma-type interferon (or immune interferon) activity.

3. A vector according to Claim 2 wherein said protein or polypeptide having the gamma-interferon activity has the amino acid sequence disclosed on page 5, lines 10-24 or the amino acid sequence disclosed from page 5, line 26 to page 6, line 13.

4. A vector according to Claim 1 wherein said protein or polypeptide coding gene includes a chemically synthesized DNA.

5. A vector according to Claim 4 wherein said chemically synthesized DNA has the base sequence disclosed on page 7 or the base sequence disclosed on page 8.

6. A vector according to Claim 1 wherein the E. coli trpA terminator function is a chemically synthesized DNA fragment.

7. A vector according to Claim 6 wherein said chemically synthesized DNA fragment is represented by the following base sequence:

$$5' \text{ TCGACAGCCCGCCTAATGAGCGGGCTTTTTTTTC } 3'$$
$$\text{GTCGGGCGGATTACTCGCCCGAAAAAAAAAGAGCC}$$

8. A process for producing a protein or polypeptide with an interferon activity or an anti-tumor activity which comprises culturing a cell transformed by a vector according to any one of Claims 1 to 7.

**Claims for the following Contracting State : AT**

1. A process for producing a protein or polypeptide with an interferon activity or an anti-tumor activity which comprises culturing a cell transformed by a vector capable of efficient expression of a gene encoding a protein or polypeptide with an interferon activity or anti-tumor activity, characterized in that the E. coli trpA terminator is linked immediately downstream from the stop codon of said gene to be expressed.

2. A process according to claim 1 wherein the protein or polypeptide has a gamma-type interferon (or immune interferon) activity.

3. A process according to claim 2 wherein said protein or polypeptide having the gamma-interferon activity has the amino acid sequence disclosed on page 5, lines 10-24 or the amino acid sequence disclosed from page 5, line 26 to page 6, line 13.

4. A process according to claim 1 wherein said protein or polypeptide coding gene includes a chemically synthesized DNA.

5. A process according to claim 4 wherein said chemically synthesized DNA has the base sequence disclosed on page 7 or the base sequence disclosed on page 8.

6. A process according to claim 1 wherein the E. coli trpA terminator function is a chemically synthesized DNA fragment.

7. A process according to claim 6 wherein said chemically synthesized DNA fragment is represented by the following base sequence:

5' TCGACAGCCCGCCTAATGAGCGGGCTTTTTTTTC 3'
GTCGGGCGGATTACTCGCCCGAAAAAAAAGAGCC

**Patentansprüche**
**Patentansprüche für folgende Vertragsstaaten : BE, CH, DE, FR, GB, IT, LI, LU, NL, SE**

1. Vektor, der zur effizienten Expression eines Gens für ein Protein oder Polypeptid mit einer Interferonaktivität oder Antitumoraktivität in der Lage ist, dadurch **gekennzeichnet,** dass der E. coli TrpA-Terminator unmittelbar stromabwärts an das Stoppcodon des zu exprimierenden Gens gebunden ist.

2. Vektor nach Anspruch 1, worin das Protein oder Polypeptid eine Interferonaktivität vom gamma-Typ (oder Immuninterferon) aufweist.

3. Vektor nach Anspruch 2, worin das Protein oder Polypeptid mit der gamma-Interferonaktivität, die auf Seite 4 offenbarte Aminosäuresequenz oder die auf Seiten 4/5 offenbarte Aminosäuresequenz aufweist.

4. Vektor nach Anspruch 1, worin das Protein oder Polypeptid codierende Gen chemisch synthetisierte DNA umfasst.

5. Vektor nach Anspruch 4, worin die chemisch synthetisierte DNA, die auf Seite 7 offenbarte Basensequenz oder auf Seite 8 offenbarte Basensequenz aufweist.

6. Vektor nach Anspruch 1, worin die E. coli TrpA-Terminatorfunktion ein chemisch synthetisiertes DNA-Fragment ist.

7. Vektor nach Anspruch 6, worin das chemisch synthetisierte DNA-Fragment durch die folgende Basensequenz dargestellt ist:

5' TCGACAGCCCGCCTAATGAGCGGGCTTTTTTTTC 3'
GTCGGGCGGATTACTCGCCCGAAAAAAAAGAGCC.

8. Verfahren zur Herstellung eines Proteins oder Polypeptids mit einer Interferonaktivität oder einer Antitumoraktivität, welches die Kultur einer durch einen Vektor nach einem der Ansprüche 1 bis 7 transformierten Zelle umfasst.

**Patentansprüche für folgenden Vertragsstaat : AT**

1. Verfahren zur Herstellung eines Proteins oder Polypeptids mit einer Interferonaktivität oder Antitumoraktivität welches die Kultur einer Zelle umfasst, die mit einem Vektor, der zur effizienten Expression eines Gens für ein Protein und ein Polypeptid mit einer Interferonaktivität oder Antitumoraktivität in der Lage ist, transformiert wurde, dadurch **gekennzeichnet,** dass der E. coli TrpA-Terminator unmittelbar stromabwärts an das Stoppcodon des zu exprimierenden Gens gebunden ist.

2. Verfahren nach Anspruch 1, worin das Protein oder Polypeptid eine Interferonaktivität vom gamma-Typ (oder Immuninterferon) aufweist.

3. Verfahren nach Anspruch 2, worin das Protein oder Polypeptid mit der gamma-Interferonaktivität, die auf Seite 4 offenbarte Aminosäuresequenz oder die auf Seiten 4/5 offenbarte Aminosäuresequenz aufweist.

4. Verfahren nach Anspruch 1, worin das Protein oder Polypeptid codierende Gen eine chemisch synthetisierte DNA umfasst.

5. Verfahren nach Anspruch 4, worin die chemisch synthetisierte DNA, die auf Seite 7 offenbarte Basensequenz oder auf Seite 8 offenbarte Basensequenz aufweist.

6. Verfahren nach Anspruch 1, worin die E. coli TrpA-Terminatorfunktion ein chemisch synthetisiertes DNA-Fragment ist.

7. Verfahren nach Anspruch 6, worin das chemisch synthetisierte DNA-Fragment durch die folgende Basensequenz dargestellt ist:

```
5' TCGACAGCCCGCCTAATGAGCGGGCTTTTTTTTC 3'
    GTCGGGCGGATTACTCGCCCGAAAAAAAAGAGCC.
```

**Revendications**
**Revendications pour les Etats contractants suivants : BE, CH, DE, FR, GB, IT, LI, LU, NL, SE**

1. Vecteur apte à l'expression efficace d'un gène codant pour une protéine ou un polypeptide ayant une activité d'interféron ou une activité antitumorale, caractérisé en ce que le terminateur TrpA de *E. coli* est soudé immédiatement en aval du codon d'arrêt dudit gène à exprimer.

2. Vecteur selon la revendication 1, dans lequel la protéine ou le polypeptide a une activité d'interféron (ou d'interféron immun) de type gamma.

3. Vecteur selon la revendication 2, dans lequel ladite protéine ou ledit polypeptide ayant l'activité d'interféron gamma comporte la séquence d'aminoacides décrite p. 6, lignes 5-32 ou la séquence d'aminoacides décrite de la page 6 ligne 33 à la page 7 ligne 21.

4. Vecteur selon la revendication 1, dans lequel ledit gène codant pour une protéine ou un polypeptide comprend un ADN synthétisé chimiquement.

5. Vecteur selon la revendication 4, dans lequel ledit ADN synthétisé chimiquement comporte la séquence de bases décrite page 8 ou la séquence de bases décrite page 9.

6. Vecteur selon la revendication 1, dans lequel la fonction de terminateur TrpA de *E. coli* est un fragment d'ADN synthétisé chimiquement.

7. Vecteur selon la revendication 6, dans lequel ledit fragment d'ADN synthétisé chimiquement est représenté par la séquence de bases suivante

```
5'-TCGACAGCCCGCCTAATGAGCGGGCTTTTTTTTC-3'
    GTCGGGCGGATTACTCGCCCGAAAAAAAAGAGCC
```

8. Procédé pour la production d'une protéine ou d'un polypeptide ayant une activité d'interféron ou une activité antitumorale, comprenant la culture d'une cellule transformée par un vecteur selon l'une quelconque des revendications 1 à 7.

**Revendications pour l'Etat contractant suivant : AT**

1. Procédé pour la production d'une protéine ou d'un polypeptide ayant une activité d'interféron ou une activité antitumorale, comprenant la culture d'une cellule transformée par un vecteur apte à l'expression efficace d'un gène codant pour une protéine ou un polypeptide ayant une activité d'interféron ou une activité antitumorale, caractérisé en ce que le terminateur TrpA de *E. coli* est soudé immédiatement en aval du codon d'arrêt dudit gène à exprimer.

2. Procédé selon la revendication 1, dans lequel la protéine ou le polypeptide a une activité d'interféron (ou d'interféron immun) de type gamma.

3. Procédé selon la revendication 2, dans lequel ladite protéine ou ledit polypeptide ayant l'activité d'interféron $\gamma$ comporte la séquence d'aminoacides décrite p. 6, lignes 5-32 ou la séquence d'aminoacides décrite de la page 6 ligne 33 à la page 7 ligne 21.

4. Procédé selon la revendication 1, dans lequel ledit gène codant pour une protéine ou un polypeptide comprend un ADN synthétisé chimiquement.

5. Procédé selon la revendication 4, dans lequel ledit ADN synthétisé chimiquement comporte la séquence de bases décrite page 8 ou la séquence de bases décrite page 9.

6. Procédé selon la revendication 1, dans lequel la fonction de terminateur TrpA de *E. coli* est un fragment d'ADN synthétisé chimiquement.

7. Procédé selon la revendication 6, dans lequel ledit fragment d'ADN synthétisé chimiquement est représenté par la séquence de bases suivante

$$5'-TCGACAGCCCGCCTAATGAGCGGGCTTTTTTTTC-3'$$
$$GTCGGGCGGATTACTCGCCCGAAAAAAAAGAGCC$$

# Fig. 1

5'-TCGACAGCCCGCCTAATGAGCGGGCTTTTTTTTTC-3'
3'-GTCGGGCGGATTACTCGCCCGAAAAAAAAAGAGCC-5'

terminator DNA Sequence

# Fig. 2

# Fig. 3

A    B

67 Kd →

43 Kd →

30Kd →

A: W3110/pIN5T4

B: W3110/pIN5T8

20 Kd →

14 Kd →

# Fig. 4

EP 0 183 272 B1

# Fig. 5

# Fig. 6-a

A : W3110/pIN5T5

B : W3110/pIN5T13

C : W3110/pIN5T15

# Fig. 6-b

A: W3110 / pIN5T5

B: W3110 / pIN5T13

C: W3110 / pIN5T15

18Kd

EP 0 183 272 B1

# Fig. 7

# Fig. 8

Fig. 9

# Fig. 10-a

A  B  C

67Kd →

43Kd →

A: W3110/pIN5T5-131
B: W3110/pIN5T13-131
C: W3110

30Kd →

20Kd →

← GIF 131

14Kd →

# Fig. 10-b

A: W3110
/pIN5T5-131

B: W3110
/pIN5T13-131

C: W3110

16Kd

EP 0 183 272 B1